# EUROPEAN PATENT APPLICATION

(11) **EP 3 482 759 A1**
(43) Date of publication of application: **15.05.2019**
(21) Application number: 17824409.1
(22) Date of filing: 31.05.2017
(51) Int. Cl.: A61K 31/255, A61K 36/8962, A61K 31/045

(54) **POISONOUS INSECT ANTIDOTE COMPOSITION**

(30) Priority: 08.07.2016 KR 20160086884
(71) Applicant: Kim, Weonju, Gwangmyeong-si, Gyeonggi-do 14288 (KR)
(72) Inventor: Kim, Weonju, Gwangmyeong-si, Gyeonggi-do 14288 (KR)
(74) Representative: Zardi, Marco
(86) International application number: PCT/KR2017/005694
(87) International publication number: WO 2018/008853

(57) **Abstract**

The present invention relates to a novel composition for neutralizing venom from venomous insects, which can effectively neutralize venom from venomous insects, particularly bees, and is harmless to the human body, so that the composition can be used with confidence by anyone without a doctor's prescription, thus being conveniently carried and then being immediately used.

## Description

### Technical Field

The present invention relates to a composition for neutralizing venom from venomous insects, and more specifically to a novel composition for neutralizing venom from venomous insects, which can effectively neutralize venom from venomous insects, particularly bees, and is harmless to the human body, thus being conveniently used with confidence by anyone.

### Background Art

There are innumerable venomous insects in nature. Among these venomous insects, bees have a sting that is an effective weapon capable of injecting bee venom in order to defend themselves from predators.

Venom from bee stings can cause pain and rashes, and in some persons, can also cause symptoms such as stomach cramps, uterine contraction, diarrhea, difficulty in breathing, shock and the like, and in severe cases, can lead to death.

For this reason, venom from bee stings should be immediately neutralized. For neutralization of the venom from bee stings, antihistamine or steroid injection is generally prescribed by a doctor in a hospital. However, since this venom-neutralizing agent should be taken after a doctor's prescription, it is difficult to achieve immediate neutralization of the venom.

### <Prior Art Document>

### <Patent Document>

(Patent document 1) Korean Patent No. 10-0854776 (August 21, 2008)

### Disclosure

### Technical Problem

The present invention is proposed to overcome the above-described problems, and an object of the present invention is to provide a novel composition for neutralizing venom from venomous insects, which can effectively neutralize venom from venomous insects, particularly bees, and is harmless to the human body, so that the composition can be used with confidence by anyone without a doctor's prescription, thus being conveniently carried and then being immediately used after being bitten by venomous insects, particularly bees.

### Technical Solution

In accordance with one feature of the present invention, there is provided a composition for neutralizing venom from venomous insects, the composition containing, as an active ingredient, either allicin or an allicin-containing juice from a plant of the family Liliaceae.

In accordance with another feature of the present invention, the composition further contains menthol or camphor, and alcohol.

In accordance with still another feature of the present invention, the plant of the family Liliaceae is at least one selected from among garlic, onion, green onion, garlic chives (*Allium tuberosum*), and wild rocambole (*Allium monanthum*).

In accordance with yet another feature of the present invention, the composition contains, based on 100 parts by weight of the composition, 2 to 5 parts by weight of the menthol or 2 to 5 parts by weight of the camphor, and 15 to 30 parts by weight of the alcohol.

### Advantageous Effects

The composition according to the present invention comprises natural components harmless to the human body, so that the composition is harmless to the human body and causes no side effects, thus being used without a doctor's prescription. Accordingly, it can be carried conveniently by anyone and can be applied to the skin immediately after being bitten by venomous insects, particularly bees. Therefore, immediate measures can be taken, and thus a dangerous situation can be prevented from occurring.

In particular, within a maximum of about 5 minutes after the composition according to the present invention has been applied to an area stung by a bee, pain, itching, swelling and the like disappear. This suggests that the composition of the present invention has an excellent effect on venom neutralization.

Furthermore, when the composition according to the present invention further contains menthol or camphor, in addition to allicin or an allicin-containing juice from a plant of the family Liliaceae, the menthol or camphor further enhances the venom neutralizing effect of the composition, and also neutralizes the unique odor of allicin or an allyl sulfide contained in the juice from the plant of the family Liliaceae. Accordingly, the composition does not give a sense of discomfort to the user, and thus has improved commercial value.

### Mode for Invention

The present invention will be described in greater detail below. The following description will be given using bees as an example of venomous insects.

Allicin is a representative component in garlic, and allicin is converted to allicin by allinase. Allicin has strong disinfecting and antibacterial effects, and exhibits increased efficacy when being used in combination with carbohydrates, lipids, proteins or the like.

Such allicin is abundantly found in plants of the family Liliaceae, including garlic, onion, green onion, garlic chives, wild rocambole and the like. Allicin that is used in the present invention is an artificially synthesized allicin or an allicin extracted from the plant of the family Liliaceae. Particularly, a juice from the plant of the family Liliaceae is preferably used as a source of allicin.

As can be seen through examples as described below, the use of a juice from a plant of the family Liliaceae can exhibit a more quick venom-neutralizing effect compared to the use of an extract from the plant of the family Liliaceae. This is estimated to be attributable to the fact that the juice from the plant of the family Liliaceae also contains a large amount of allyl sulfide in addition to allicin, and, when the juice from the plant of the family Liliaceae is used without change, allyl sulfide contained in the juice, together with allicin, can also exhibit a venom-neutralizing effect, unlike the use of allicin extracted from the juice.

Meanwhile, the composition according to the present invention may further contain menthol or camphor, and alcohol.

Menthol is a monoterpene alcohol consisting of a single ring structure, and is obtained by steam distillation of the leaf or stem of mint. Menthol is a colorless needle-shaped crystal having a unique cool feeling, and hardly dissolves in water, but easily dissolves in ethanol, ether or the like. It is used as a refreshing agent or a pain relieving agent and taken internally or applied externally.

Camphor (chemical formula: C₁₀H₁₆O) is a monoterpene ketone and is obtained by steam distillation of pieces of camphor wood carving. Such camphor has a refreshing taste and may also be used as a pain relieving agent or a preservative.

In the case in which such menthol or camphor is used in the present invention, when the composition is applied to the skin, such menthol or camphor gives a cool feeling and a refreshing feeling by lowering skin fever, and thus can exhibit a pain-relieving effect and helps subside swelling. In addition, such components neutralize the unique odor of allicin or allyl sulfide, and thus the composition has improved commercial value without giving a sense of discomfort to the user.

Furthermore, the alcohol that is used in the present invention functions as a solvent that dissolves menthol or camphor, and also functions to prevent the deterioration of allicin or the juice from the plant of the family Liliaceae. Accordingly, the alcohol that is used in the present invention prevents the deterioration of the composition of the present invention to thereby improve the preservability of the composition. In addition, the alcohol improves the integrity of the composition of the present invention. It will be apparent that as described above, camphor has a preservative property and serves as a preservative, and thus it also improves the integrity of the composition of the present invention.

Preferably, the composition of the present invention contains, based on 100 parts by weight of the composition, 2 to 5 parts by weight of menthol or camphor and 15 to 30 parts by weight of alcohol. If the content of menthol or camphor in the composition is less than 2 parts by weight, the effect of menthol or camphor may be insignificant, and if the content of menthol or camphor is more than 5 parts by weight, the content of alcohol should also be increased, and thus the relative content of active ingredients, including allicin, may undesirably be reduced.

Furthermore, when the content of menthol or camphor is in the above-described range, the content of alcohol in the composition is preferably 15 to 30 parts by weight. If the content of alcohol in the composition is less than 15 parts by weight, menthol or camphor may not be appropriately dissolved. In contrast, if the content of alcohol in the composition is more than 30 parts by weight, the integrity of the composition may be improved, but the relative content of active ingredients, including allicin, may undesirably be reduced, so that the venom neutralizing effect of the composition is significantly reduced. Accordingly, this case is not desirable.

### Examples 1 to 5

Each of the venom-neutralizing compositions shown in Table 1 below was applied to bee-stung areas in 10 persons, and the venom-neutralizing effects of the compositions were monitored. The venom-neutralizing effect can be verified by the disappearance of pain, itching and swelling, and the results are listed in Table 2 below:

**[Table 1]**

| Components | Example 1 | Example 2 | Example 3 | Example 4 | Example 5 |
|---|---|---|---|---|---|
| Allicin | 100 | 80 | - | - | - |
| Garlic juice | - | - | 80 | - | - |
| Onion juice | - | - | - | 80 | - |
| Green onion juice | - | - | - | - | 80 |
| Menthol | - | 18 | 18 | 8 | 18 |
| Alcohol | - | 2 | 2 | 2 | 2 |

**[Table 2]**

| | | Example 1 | Example 2 | Example 3 | Example 4 | Example 5 |
|---|---|---|---|---|---|---|
| Pain | Disappeared within 3 minutes | 9 | 9 | 9 | 8 | 7 |
| | Disappeared after about 4 minutes | 1 | 1 | 1 | 1 | 2 |
| | Disappeared after about 5 minutes | - | - | - | 1 | 1 |
| Itching | Disappeared within 3 minutes | 6 | 7 | 8 | 7 | 5 |
| | Disappeared after about 4 minutes | 2 | 2 | 2 | 3 | 5 |
| | Disappeared after about 5 minutes | 2 | 1 | - | - | - |
| Swelling | Disappeared within 3 minutes | 7 | 7 | 9 | 8 | 7 |
| | Disappeared after about 4 minutes | 2 | 1 | 1 | 2 | 3 |
| | Disappeared after about 5 minutes | 1 | 2 | - | - | - |

**[Table 3]**

| Overall evaluation | Example 1 | Example 2 | Example 3 | Example 4 | Example 5 |
|---|---|---|---|---|---|
| Very good | 8 | 8 | 9 | 9 | 7 |
| Good | 2 | 3 | 1 | 1 | 3 |
| Moderate | - | - | - | - | - |
| Poor | - | - | - | - | - |

As can be seen in Tables 2 and 3 above, within about 5 minutes after the composition according to the present invention was applied to the bee-stung areas, pain and itching disappeared, and subside also subsided.

Furthermore, when allicin was used along with menthol and alcohol, itching and swelling subsided faster than those when allicin was used alone. Particularly, it was verified that the use of garlic juice or onion juice exhibited a better effect than the use of allicin.

This effect is estimated to be attributable to allicin and the allyl sulfide contained in the garlic juice or onion juice. Moreover, in Examples 3, 4 and 5, it was observed that the venom-neutralizing effect was the highest in the composition of Example 3, which contains garlic juice, and was higher in the order of the composition of Example 4, which contains onion juice, and the composition of Example 4, which contains green onion juice. This is estimated to be attributable to the fact that the contents of allicin and allyl sulfide in garlic juice and onion juice are higher than those in green onion juice.

In addition, as can be seen in Table 4 above, in response to the overall evaluation, almost all of the test participants answered that the venom-neutralizing effect of the composition of the present invention was very good.

In particular, 9 of the 10 test participants answered that the venom-neutralizing effects of the compositions of Examples 3 and 4, which contained a mixture of garlic juice or onion juice with alcohol, were very good.

Although the present invention has been described using the neutralization of venom from bees as an example, the venom-neutralizing composition according to the present invention is not applied only to neutralization of venom from bees, but may be used for neutralization of venom from various venomous insects.

## Claims

1. A composition for neutralizing venom from venomous insects, the composition containing an allicin-containing juice from a plant of a family Liliaceae.

2. The composition of claim 1, further containing menthol or camphor, and alcohol.

3. The composition of claim 1, wherein the plant of the family Liliaceae is at least one selected from among garlic, onion, green onion, garlic chives, and wild rocambole.

4. The composition of claim 2, containing, based on 100 parts by weight of the composition, 2 to 5 parts by weight of the menthol or the camphor, and 15 to 30 parts by weight of the alcohol.
